# EUROPÄISCHE PATENTSCHRIFT

(11) **EP 0 965 334 B1**
(45) Veröffentlichungstag und Bekanntmachung des Hinweises auf die Patenterteilung: **13.03.2002**
(21) Anmeldenummer: 99111588.2
(22) Anmeldetag: 09.08.1996
(51) Int. Cl.: A61K 9/00, A61K 31/02

(54) **Intubations-Flüssigkeits-Beatmungsmittel**
Liquid respiratory product for intubation
Produit liquide respiratoire pour intubation

(30) Priorität: 29.09.1995 DE 19536504
(43) Veröffentlichungstag der Anmeldung: 22.12.1999
(62) Teilanmeldung aus: 96927711.0
(73) Patentinhaber: Meinert, Hasso, 89231 Neu-Ulm (DE)
(72) Erfinder: Meinert, Hasso, 89231 Neu-Ulm (DE)
(74) Vertreter: Nöth, Heinz, Dipl.-Phys.

(56) Entgegenhaltungen:
- US-A- 4 173 654

## Beschreibung

Es ist bekannt, daß Sauerstoff-gesättigte flüssige Perfluorcarbone, z.B. Perfluordekalin, bei bestimmten chirurgischen Eingriffen derartig verwendet werden, daß über einen bis zur Luftröhre eingeführten Tubus dieses Perfluorcarbon direkt in die Lunge und damit ins Alveolarsystem eingebracht wird. Die Respiration wird dabei über das O₂- angereicherte bzw. gesättigte Perfluorcarbon gewährleistet, ein Zusammenfallen der Lunge wird gleichzeitig verhindert. Bei der bekannten Flüssigatmung sind sowohl bei chirurgischen Eingriffen als auch im Falle von Tauchern Schwierigkeiten insofern gegeben, als durch die hohe Dichte des Perfluorcarbons (ca. 1,8 - 2,0 g/cm³) die Atmung bzw. der Transport des O₂- bzw. CO₂-transportierenden Mediums seitens des geschwächten oder gestressten Körpers erschwert wird.

Aus [8] ist die Synthese von Perfluorohydrocarbonen bekannt, bei denen endständige Perfluoralkyl-Einheiten über ein tertiäres, dreifach R_{F}-substituiertes C-Atom direkt oder über einen -(CH₂)--Spacer an aliphatische oder olefinische Gruppen gebunden sind. Dabei sind stets zwei der R_{F}-Substituenten des endständigen tertiären C-Atoms CF₃-Gruppen. Die bekannten Verbindungen werden zur Herstellung von Blutersatzstoffen in Form von wässrigen Emulsionen oder als Kühlflüssigkeiten für die Elektronik verwendet. Ferner sollen die bekannten Verbindungen zur Fiüssigatmung und extrazirkulären Lungenwäsche bei Rauchinhalation und anderen Lungenerkrankungen verwendet werden.

Gemäß Patentanspruch 1 wird zur Herstellung eines Intubations-Flüssigkeits-Beatmungsmittel die Verwendung eines semifluorierten Alkans und gemäß Patentanspruch 5 wird eine Vorrichtung zur Flüssigatmung mit einem O₂- bzw. CO₂-transportierendem Medium aus einem semifluorierten Alkan vorgeschlagen.

Hierdurch wird eine erleichterte Respiration und auch die Entfaltung von Atelektase-Lungen gewährleistet. Bei Lungenobstruktion können diese Intubations-Flüssigkeit-Beatmungsmittel zur Entfaltung der kollabierten Lunge sowie bei der bislang noch nicht genannten, der Taucheratmung analogen Beatmung von Raumfahrern verwendet werden.

Bei der den Perfluorcarbonen analogen hohen Löslichkeit von Gasen (R_{F}R_{H} bzw. R_{F}R_{H}R_{F} : ca. 45-52 vol.% O₂, 145-150 vol.% CO₂), welche auch die Löslichkeit von Stickoxiden und Narkosegas beinhaltet, beträgt die Dichte der vorgeschlagenen flüssigen semifluorierten Alkane vorteilhafterweise nur ca. 1,1-1,7 g/cm³ (25°C).

Für die genannten Methoden der Instillations- bzw. Flüssigbeatmung sind die Vorteile bei der Verwendung der vorgeschlagenen semifluorierten Alkane gegenüber den ansonsten hierfür nur anwendbaren Perfluorcarbonen somit eindeutig in einer erleichterten Respiration gegeben. Diese sogenannte Liquid Ventilation kann als vollständige oder partielle Flüssigatmung durchgeführt werden.

Diese Flüssigatmung ist mit Hilfe der semifluorierten Alkane auch bei Tauchern möglich. Dabei kann über den Tubus das in die Lunge gebrachte O₂- bzw. CO₂-transportierende Medium über ein Kreislaufsystem mittels eines Oxygenerators ständig mit O₂ angereichert und von CO₂ mittels eines entsprechenden CO₂-Rückhaltesystems befreit werden. Ein derartig versorgter Taucher muss nicht mehr Luft bzw. Sauerstoff über Pressluftflaschen einatmen und verbrauchte Luft über ein Ventilsystem ausatmen. Die Ventilgeräusche und die austretenden Gas- bzw. Luftblasen machen den Taucher über sensible Sonarsysteme auffindbar.

Die zum Einsatz kommenden semifluorierten Alkane haben die allgemeinen Formeln

R_{F}R_{H} und R_{F}R_{H}R_{F}

wobei R_{F} eine lineare oder verzweigte Perfluoralkyl-Gruppe und R_{H} eine lineare oder verzweigte, gesättigte Kohlenwasserstoff-Alkyl-Gruppe ist, die unverzweigten semifluorierten Alkane die Formeln

F(CF₂)ₙ(CH₂)ₘH

F(CF₂)ₙ(CH₂)ₘ(CF₂)ₙF

mit n = 3 - 20
m = 3 - 20 besitzen, die verzweigten semifluorierten Alkane innerhalb der Perfluoralkyl-Gruppen -FCX- -Einheiten
mit X = C₂F₅, C₃F₇ oder C₄F₉
sowie innerhalb der Kohlenwasserstoff-Alkyl-Gruppen auch - HCY- -Einheiten
mit Y = C₂H₅ , C₃H₇ oder C₄H₉
enthalten, und stets die Gesamtzahl der Kohlenstoffatome im Perfluoralkyl-Teil bzw. in den Perfluoralkyl-Teilen in den Grenzen von n = 3 - 20 und im Kohlenwasserstoff-Alkyl-Teil die Zahl der Kohlenstoffatome in den Grenzen von m = 3 - 20 bleibt.

Innerhalb einer Perfluoralkyl-Kette kann auch eine -CX₂--Gruppe, sowie innerhalb einer Alkyl-Kette auch eine -CY₂--Gruppe enthalten sein. Dabei ist im Falle der -CX₂--Gruppe das tertiäre Kohlenstoffatom nicht direkt mit der benachbarten Kohlenwasserstoff-Alkylgruppe verbunden.

Endständig im Molekül kann anstelle der Perfluoralkyl-Gruppe F₃C- auch eine FCX₂- oder F₂CX- -Gruppe
mit X = C₂F₅, C₃F₇ oder C₄F₉
gebunden sein und ebenso kann endständig im Molekül anstelle der Alkyl-Gruppe H₃C- auch eine HCY₂- oder H₂CY- -Gruppe
mit Y = C₂H₅, C₃H₇ oder C₄H₉
gebunden sein.

Stets aber bleibt im Falle aller Isomeren, d. h. linearen oder verzweigten semifluorierten Alkane, die Gesamtzahl der Kohlenstoffatome im Perfluoralkyl-Teil bzw. in den Perfluoralkyl-Teilen wie vorgegeben in den Grenzen von n = 3 - 20, auch bleibt im Alkyl-Teil die Zahl der Kohlenstoffatome in den vorgegebenen Grenzen von m = 3 - 20.

Bei diesen semifluorierten Alkanen, kurz als Diblock-Verbindungen R_{F}R_{H} oder Triblock-Verbindungen R_{F}R_{H}R_{F} bezeichnet, kann eine intramolekulare HF-Abspaltung unter Ausbildung fluorolefinischer Doppelbindungen nicht stattfinden. Im Gegenteil, die geschlossene Kohlenwasserstoff-Alkan-Gruppierung wirkt noch bindungsverstärkend auf die ohnehin sehr festen C-F-Bindungen im Perfluoralkyl-Teil der jeweiligen Verbindung.

Die vorgeschlagenen Verbindungs-Typen sind analog den Perfluorcarbonen (Verbindungen, die nur aus Kohlenstoff-Fluor-Bindungen bestehen) chemisch, physikalisch und physiologisch inert und somit untoxisch.

Diese semifluorierten Alkane werden als medizinische Hilfsmittel zur Instillations- bzw. Flüssigbeatmung bei der vollständigen und partiellen Liquid Ventilation, bei chirurgischen Eingriffen bzw. im medizinischen Notfall und zur erleichterten Respiration von Tauchern verwendet.

Ferner eignen sich die linearen oder verzweigten semifluorierten Alkane, insbesondere wenn sie einen relativ hohen Alkylanteil, -(CH₂)ₙ- bzw. -(CH₂)ₙH bzw. deren R_{H}-substituierte Isomere, besitzen, als Lösungsmittel von Medikamenten.

Die zum Einsatz kommenden semifluorierten Diblock- oder Triblock-Alkane sind farblose Flüssigkeiten. Sie werden nicht von starken Säuren oder Laugen oder Oxidationsmitteln oder Nukleophilen angegriffen, ebenso wenig findet ein metabolischer oder katabolischer Angriff statt.

Analog den Perfluorcarbonen und den Kohlenwasserstoffen sind die semifluorierten Alkane in Wasser kaum bis nicht löslich.

Jedoch lassen sich die vorgeschlagenen semifluorierten Alkane mittels wirksamer nichtionischer Tenside (Fluortenside, verbinden mit fluorophilem Kopf und hydrophilem Schwanz, Ethylenoxid-Propylenoxid-Blockpolymere, Pluronics® oder Phospholipide, wie Eilezetin oder Sojalezetin, u.a.) oder ionischer Tenside unter Zuhilfenahme von physikalischen Emulgierungsverfahren (Ultraschall- bzw. Gaulin-Homogenisatoren) in teilchenstabilen Emulsionen mit Partikelgrössen von ca. 100-300 nm analog [1] überführen. Dabei sind die Diblock-Verbindungen besser emulgierbar als die Triblock-Verbindungen.

Zum Unterschied zu den Perfluorcarbonen sind die semifluorierten Alkane, unabhängig vom jeweiligen RF- bzw. R_{H}-Anteil, sowohl in Perfluorcarbonen und Derivaten von Perfluorcarbonen (höhermolekulare perfluorierte Ether, Hostinert®, Fomblin® u.a.) [1-4] als auch in Kohlenwasserstoffen und deren Derivaten und dabei Verbindungen mit höheren Alkylgruppen-Anteilen (z.B. flüssige Paraffine, Silikonöle, Fettsäure-Ester u.a.) löslich. Dabei steigt mit steigendem Perfluoralkyl-Anteil die Löslichkeit in den Fluorcarbon-Systemen, während mit steigendem Alky-Anteil die Löslichkeit in den Kohlenwasserstoff-Systemen zunimmt, und umgekehrt.

Im Gegensatz zu den Perfluorcarbonen (Dichten 1,8 - 2,0 g/cm³) liegen die Dichten der semifluorierten Alkane mit Werten zwischen 1,1 bis 1,7 g/cm³ wesentlich niedriger, dadurch bedingt, daß diese Moleküle einen hohen Anteil an Kohlenwasserstoff-Gruppierungen, enthalten.

Die semifluorierten Diblock- oder Triblock-Alkane werden durch Umsetzung von Perfluoralkyliodiden mit Alkenen oder α,ω-Dienen, nach HI-Eliminierung und abschließender Hydrierung mittels H₂/Raney-Nickel im Hochdruckverfahren [5] oder durch Hydrierung mittels Platin-Kontakt [6] oder mittels Tributylzinnhydrid [4] gewonnen (s. Anhang: Beispiele für Synthesen semifluorierter Diblock- und Triblockalkane).

Die dabei erhaltenen Produkte für die vorgeschlagenen Einsatzgebiete können in bevorzugter Weise noch hochgereinigt werden. Dazu werden die semifluorierten Alkane analog [7] zunächst mit saurer Permanganatlösung behandelt, danach werden sie mit einem Gemisch von starker wäßriger Kalilauge (4-8 n), CaO bzw. BaO, und einem nukleophilen Agenz (z. B. sekundäres Amin) bei 150-180°C ca. 72 Stunden lang am Rückfluß erhitzt oder autoklaviert. Das Umsetzungsprodukt wird abschließend mittels Scheidetrichter von der wässrigen alkalischen Phase, die gegebenenfalls noch Alkohol enthält, und der Aminphase abgetrennt, mehrmals nacheinander mit verdünnter Mineralsäure, NaHCO₃-Lösung, destilliertem Wasser, wasserfreiem Na₂SO₄ und wasserfreiem CaCl₂ behandelt und über eine leistungsfähige Kolonne fraktioniert destilliert.

Die so behandelten semifluorierten Alkane sind gemäß IR-, ¹H-NMR, ¹⁹F-NMR-, GC-/MS-Spektroskopie frei von Gruppierungen, die unter intramolekularer HF-Eliminierung zur Bildung toxischer olefinischer Nebenprodukte führen könnten.

Als quantitatives Verfahren zur Bestimmung von Gruppierungen, die zur intramolekularen HF-Abspaltung oder zum Austausch eines Fluoratoms, falls dieses in einer -CHF--Gruppierung gebunden wäre, mittels eines nukleophilen Agenz führen können, eignet sich nach [7] die Bestimmung von ionisierbarem Fluorid bei der Reaktion des Probenmaterials mit Hexamethylendiamin in Nonan bzw. Dekan durch mehrstündiges Erhitzen bei 120 - 150°C, wobei eventuell freigesetztes Fluorid mittels einer ionensensitiven Elektrode erfasst wird. Nach dem Reinigungsverfahren waren demzufolge keine Fluoridionen mehr nachweisbar (Erfassungsgrenze für die Fluoridkonzentration ≤ 10⁻⁵ mol · l⁻¹).

Die hochgereinigten semifluorierten Alkane zeigen keine Proliferationshemmung bezüglich DNS- und Proteinsynthese an HeLa- oder Molt4- oder HEP₂-Zellkulturen. Damit sind die genannten semifluorierten Alkane für medizinische, pharmazeutische und biologische Zwecke direkt einsetzbar.

Wie eingangs beschrieben, lassen sich die genannten semifluorierten Alkane, vorzugsweise die Diblock-Verbindungen R_{F}R_{H}, sehr leicht mittels biokompatibler Emulgatoren (natürliche Phospholipide, wie Soja- oder Eilezethin oder synthetisch herstellbare Lezithine, oder Phospholipid-Mischungen mit einem Anteil von 60-98% an Phosphatidylcholin oder Ethylenoxid-Propylenoxid-Blockpolymer, Pluronic®, u.a.) unter Zuhilfenahme physikalischer Emulgiermethoden (Ultraschall- bzw. Gaulin-Homogenisator, Ultraturrax-Dispergiergerät) zu stabilen, wässrigen Emulsionen des o/w- und w/o-Types mit asymetrischen lamellaren Aggregaten verarbeiten.

Von Vorteil ist die Verwendung der leicht beweglichen, flüssigen semifluorierten Alkane, z. B. C₆F₁₃C₈H₁₇ (Kp 222°C) , C₄F₉C₅H₁₁ (Kp 134°C) oder C₃F₇C₄H₈C₃F₇ (Kp 180 °C).

Bei der den Perfluorcarbonen analogen Gaslöslichkeit (R_{F}R_{H} bzw. R_{F}R_{H}R_{F} : ca. 45-52 vol.% O₂, 145-150 vol.% CO₂) beträgt die Dichte der vorgeschlagenen flüssigen semifluorierten Alkane nur 1,1-1,7 g/cm³ (25 °C).

Für die genannten Methoden der Instillations- bzw. Flüssigbeatmung sind die Vorteile bei der Verwendung der vorgeschlagenen semifluorierten Alkane gegenüber den ansonsten hierfür nur anwendbaren Perfluorcarbonen somit eindeutig in einer erleichterten Respiration gegeben.

### Literatur

[1] H. Meinert, A. Knoblich, Biomat. Art. Cells & Immob. Biotech., 21 (1993) 583
[2] H. Meinert, Fluorine in Medicine in the 21st Century, Manchester (1994), paper 23
[3] R.J. Twieg et al., Macromolecules *18* (1985) 1361
[4] J. Höpken et al., Macromol. Chem. *189* (1988) 911
[5] K. von Werner, DE 39 25 525 A1 (1989)
[6] Organikum, Autorenkollektiv, Dtsch. Verlag der Wissenschaften, Berlin (1977) 363
[7] H. Meinert DE 42 05 341 A1 (1992) / WO 93/16974 A1 (1993)
[8] K.V. Scherer US-A-4,173,654

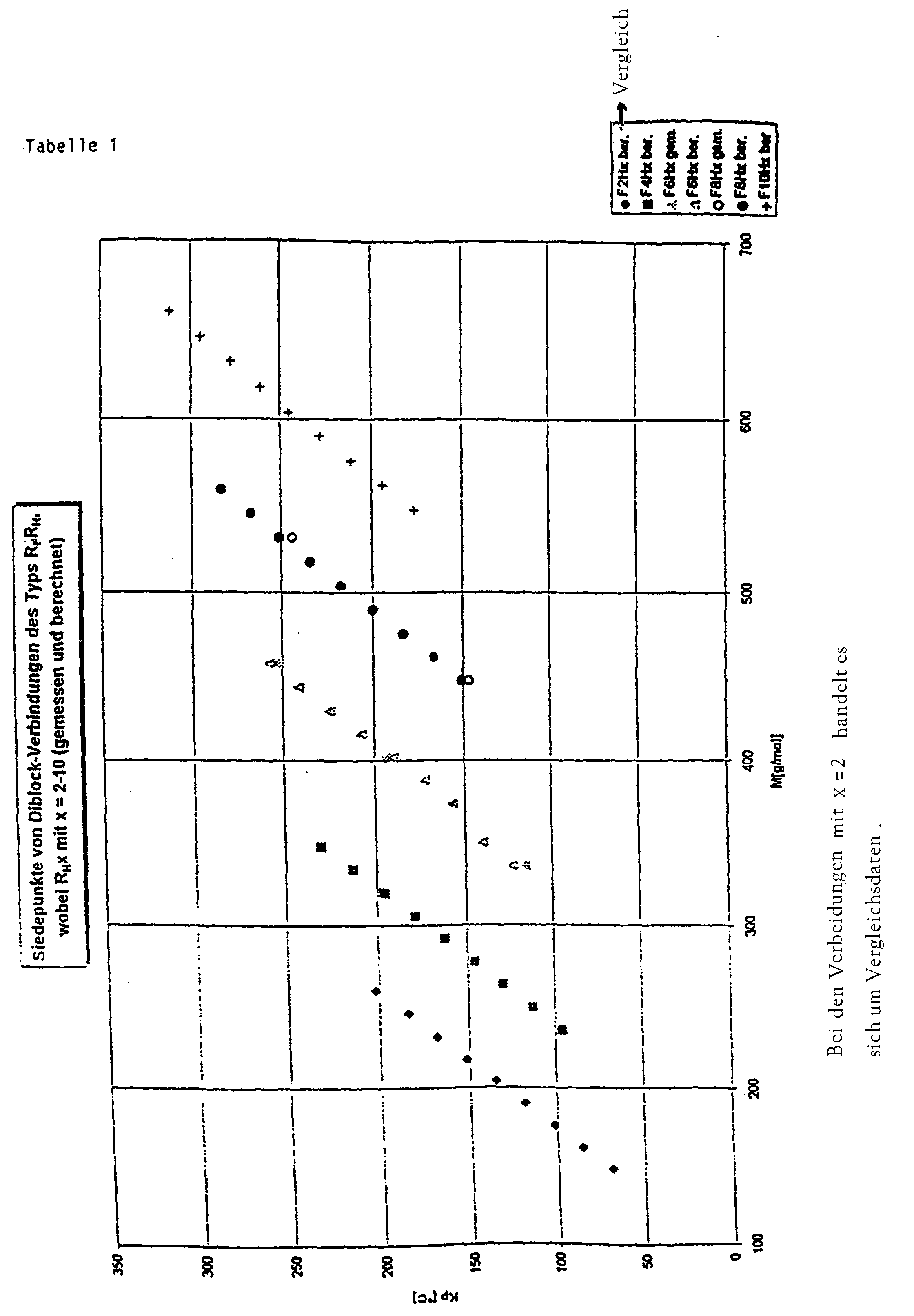

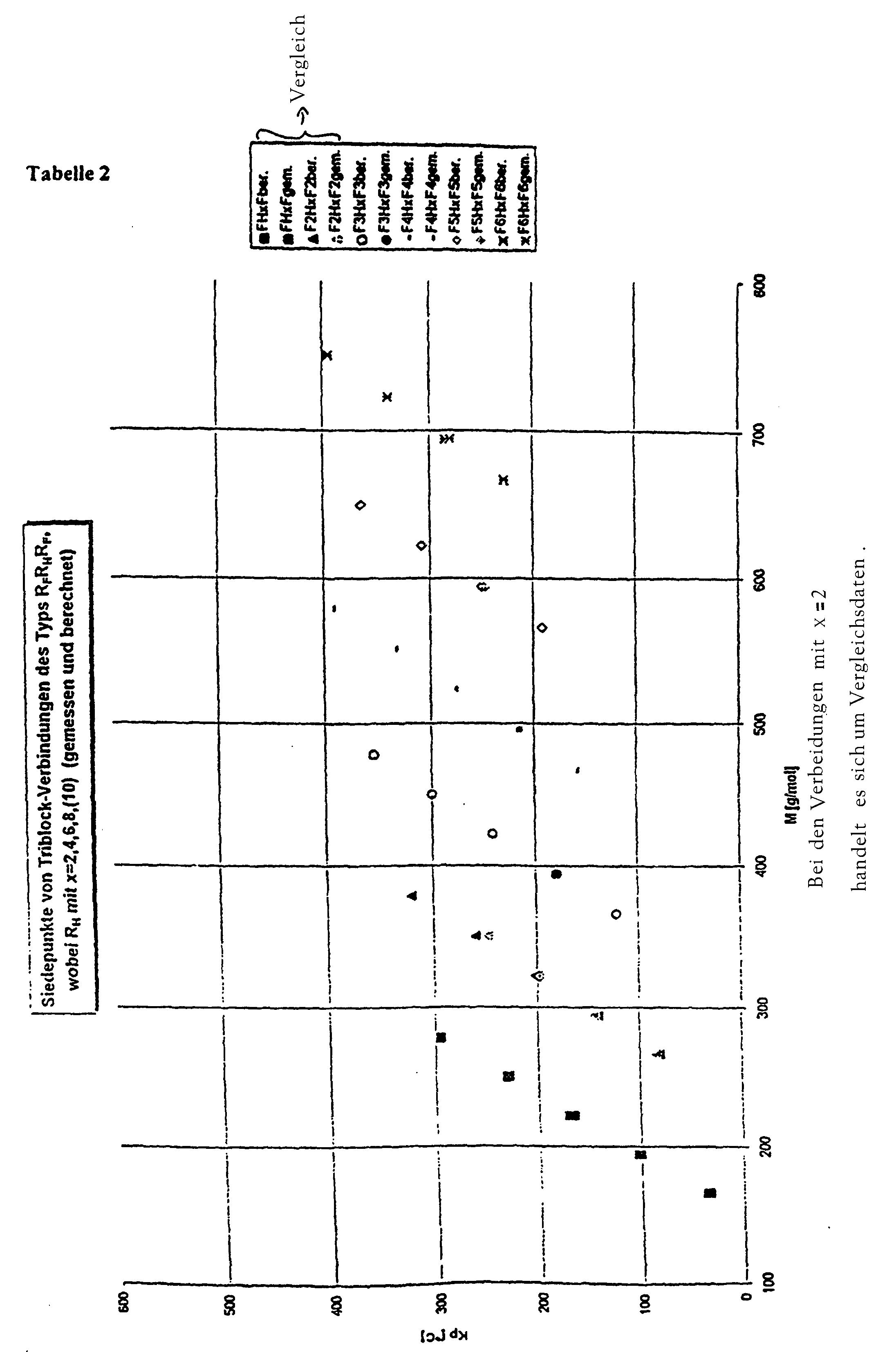

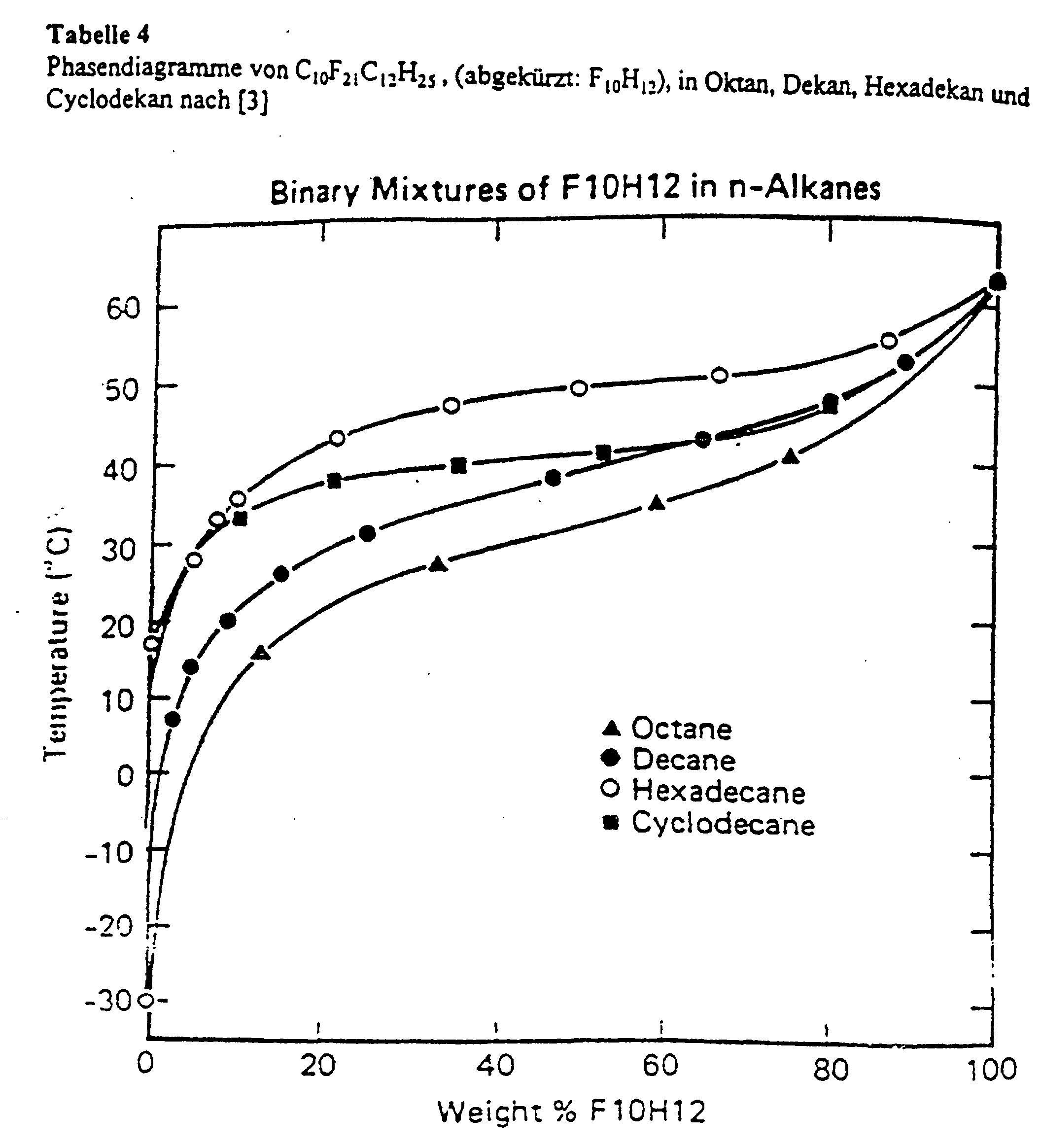

### Beispiele für Synthesen semifluorierter Diblock- und Triblock-Alkane

### Darstellung semifluorierter Diblock- und Triblock-Alkane

### Beispiel 1

Nach [5] werden in einem Vierhalskolben mit 250 cm³ Inhalt, der mit Tropftrichter (mit Umgang zum Druckausgleich), Flügelrührer, Thermometer und Rückflußkühler mit aufgesetztem Rückschlagventil ausgestattet ist, unter Argon 1,82 g (0,075 mol) Magnesiumspäne in 10 cm³ wasserfreiem Di-n-propylether vorgelegt und mit einigen Tropfen Methyliodid unter leichtem Erwärmen aktiviert. Die Temperatur wird auf 80 °C erhöht, während eine Mischung aus 23,7 g (0,05 mol) C₆F₁₃CH₂CH₂I und 60 cm³ wasserfreiem Di-n-propylether innnerhalb 1 Stunde unter kräftigem Rühren zugetropft wird. Anschließend wird die Mischung 9 Stunden unter Rückfluß gerührt, dann auf 10 °C abgekühlt, mit 100 cm³ 5gew.-%iger wäßriger Salzsäure hydrolysiert, bis sich das überschüssige Magnesium abgelöst hat. Nun wird die Etherphase abgetrennt und mit einem Rotationsverdampfer aufkonzenteriert. Der so erhaltene ölige Rückstand wird mit 30 cm³ Chloroform versetzt und 1 Stunde bei 0 °C stehengelassen. Das aus der Mischung abgeschiedene feste Produkt wird abgesaugt und im Exsikator getrocknet. Es werden 11,0 g C₆F₁₃(CH₂)₄C₆F₁₃ als fast farblose Kristalle erhalten, die einen Schmelzpunkt von 48 °C aufweisen. Die Ausbeute beträgt 63,2 % des theoretischen Wertes.
Vermittelts Kernresonanz-Spektralanalyse werden folgene Werte ermittelt (in CDCl3-Lösung, mit Tetramethylsilan als internen Standard):
¹H-NMR: 2,11 ppm (CF₂CH₂), 1,72 ppm (CH₂CH₂)

### Beispiel 2

Nach [5] wird verfahren wie in Beispiel 1 angegeben, wobei folgende Stoffe eingesetzt werden:
1,82 g (0,075 mol) Magnesiumspäne
28,7 g (0,05 mol) C₈F₁₇CH₂CH₂I
70 cm³ wasserfreier Di-n-propylether

Nach der Hydrolyse mit verdünnter Salzsäure wird das Gemisch filtriert, das Rohprodukt mit Wasser gewaschen und im Exsikator getrocknet, anschließend aus Chloroform umkristallisiert und im Vakuum getrocknet. Es werden 14,9 g farbloser Blättchen der Verbindung C₈F₁₇(CH₂)₄C₈F₁₇ erhalten, die einen Schmelzpunkt von 92 bis 93 °C aufweisen. Die Ausbeute beträgt 66,8 % des theoretischen Wertes.
Bei der Kernresonanz-Spektralanalyse werden folgende Werte ermittelt:
¹H-NMR: 2,12 ppm [CF₂CH₂, ³J(HF) = 18,2 Hz], 1,73 ppm (CH₂CH₂)
¹³C-NMR: 31,17 ppm [CF₂CH₂, ²J(CF) = 22,6 Hz], 20,37 ppm (CH₂CH₂)

### Beispiel 3

Nach [5] wird verfahren wie in Beispiel 2 beschrieben, jedoch werden nach der Aktivierung der Magnesiumspäne mit Methyliodid dem Reaktionsansatz 93,6 mg (0,143 mmol) der Verbindung [(C₆H₅)₃P]₂CoCl₂ als Katalysator zugesetzt. Nach dem Umkristallisieren des Reaktionsproduktes aus Chloroform und Vakuumtrocknung, wie in Beispiel 2 beschrieben, werden 16,6 g der Verbindung C8F17(CH2)4C8F17 erhalten, die einen Schmelzpunkt von 92 bis 93 °C aufweist. Die erhaltene Menge entspricht 74,1 % des theoretischen Wertes.
Die Kernresonanz-Spektralanalyse ergibt die gleichen Werte, wie unter Beispiel 2 angegeben.

### Beispiel 4

Es wird wiederum gearbeitet, wie in Beispiel 2 angegeben, jedoch werden nach der Aktivierung der Magnesiumspäne dem Reaktionsansatz 2,2 g [0,005 mol = 10 Mol-%, bezogen auf eingesetzte Verbindung der Formel C₈F₁₇CH₂CH₂I] von der Verbindung C₈F₁₇CH = CH₂ zusammen mit dem Gemisch aus wasserfreiem Di-n-propylether und der Verbindung C₈F₁₇CH₂CH₂I zugesetzt.
Nach Umkristallisieren des Reaktionsproduktes aus Chloroform und Vakuumtrocknung werden 17,5 g der Verbindung C₈F₁₇(CH₂)₄C₈F₁₇ erhalten, die einen Schmelzpunkt von 92 bis 93 °C aufweist. Die erzeugte Menge entspricht 78,1 % des theoretischen Wertes.
Durch Kernresonanz-Spektralanalyse werden die gleichen Werte ermittelt, wie unter Beispiel 2 angegeben.
Nach Filtration des Rohproduktes wird aus dem Filtrat die Di-n-propylether enthaltende Phase abgetrennt und mit Natriumsulfat getrocknet. Die gaschromatographische Analyse der so erhaltenen Etherlösung ergibt einen Gehalt von 2,31 g der Verbindung C₈F₁₇CH = CH₂. Die ursprünglich eingesetzten 2,2 g dieser Verbindung bleiben offensichtlich unverändert, sie wirken also als Katalysator, darüber hinaus entsteht eine geringe Menge der gleichen Verbindung während der Umsetzung mit Magnesium aus dem Perfluoroctylethyliodid.

### Beispiel 5

Nach [5] wird gearbeitet, wie in Beispiel 1 beschrieben, wobei folgende Stoffe eingesetzt werden:
1,82 g (0,075 mol) Magnesiumspäne
30,1 g (0,05 mol) C₈F₁₇(CH₂)₄I
70 cm³ wasserfreier Di-n-propylether

Nach der Hydrolyse mit verdünnter Salzsäure wird das Gemisch filtriert, das abgefilterte Rohprodukt mit Wasser gewaschen und im Exsikator getrocknet. Nach dem Umkristallisieren aus Chloroform und Trocknen im Vakuum werden 14,3 g der Verbindung C₈F₁₇(CH₂)₈C₈F₁₇ als farblose Blättchen erhalten, die einen Schmelzpunkt von 84,5 °C aufweist. Die erzeugte Menge entspricht 60,1 % des theoretischen Wertes.

Bei der Kernresonanz-Spektralanalyse werden folgende Werte ermittelt:
Zuordnung: C₈F₁₇-CH₂-CH₂-CH₂-CH₂-CH₂-CH₂-CH₂-CH₂-C₈F₁₇
¹H-NMR: (1)1(8) 2,01 ppm, (2)/(7) 1,62 ppm, (3)/(4)/(5) und (6) 1,38 ppm
¹³C-NMR: (1)/(8) 31,21 ppm, [²J(CF) = 22,1 Hz], (2)/(7) 20,34 ppm, (3)/(4)/(5) und (6) 29,17 ppm

### Beispiel 6

Nach [5] wird gearbeitet wie in Beispiel 5 beschrieben, jedoch werden anstelle der Verbindung C₈F₁₇CH = CH₂ nach Aktivierung der Magnesiumspäne 1,2 g [0,0025 mol = 5 Mol-%, bezogen auf eingesetzte Verbindung C₈F₁₇(CH₂)₄] von der Verbindung C₈F₁₇(CH₂)₂CH = CH₂ als Katalysator zugesetzt. Nach der Aufarbeitung werden 16,2 g der Verbindung C₈F₁₇(CH₂)₈C₈F₁₇ erhalten, die einen Schmelzpunkt von 84,5 °C aufweist. Die erzeugte Menge entspricht 68,2 % der theoretisch zu erhaltenden Ausbeute.

### Beispiel 7

### Darstellung semifluorierter Diblock-Alkane nach [1]

2 mmol Perfluoralkylhalogenid und 4 mmol Alken( 1) wurden in 15 ml Octan gelöst, mit Argon entgast und auf 90 °C erhitzt. Anschließend wurden 150 mg Azo-iso-butyronitril innerhalb von 30 min verteilt auf mehrere Portionen zugegeben. Dabei trat eine leichte Gelbfärbung der Lösung auf. Anschließend wurde das Gemisch destillativ getrennt. Die gewünschten Verbindungen der Art R_{F}-CHI-CH₂-R_{F} ließen sich bei einem verminderten Druck von < 0.5 mbar destillieren. Die Ausbeute betrug 85 bis 90 % bezogen auf die eingesetzte Menge Perfluoralkylhalogenid bei den Iodiden und 22 % bei den Bromiden.

### Reduktion der Perfluoralkylalkylhalogenide

6.6 mmol Pefluoralkylalkylhalgoenid wurden in 15 ml Diethylether gelöst und 5 ml Essigsäure zugegeben. Das Gemisch wurde auf 50 °C erhitzt und langsam 4 mmol Zink zugegeben. Nach dem Abkühlen wurde Wasser zugegeben und die Phasen getrennt. Die organische Phase wurde getrocknet und destilliert. Dabei ließen sich bis zu 68 % eines Gemisches aus semifluorierten Alkanen und Alkenen (5:1) und etwa 10 % des Dimerierungsproduktes isolieren.

### Beispiel 8

### Darstellung semifluorierter Triblock-Alkane nach [1]

Zu 30 n-Dibuthylether und 4 g Magnesium wurden bei 60 °C 4 g C₆F₁₃C₂H₃ zugegeben, die Temperatur auf 120 °C erhöht. Danach wurden 40 g C₆F₁₃C₂H₄I gelöst in n-Dibuthylether zugegeben. Nach ca. 90 Minuten hatte die Lösung eine tiefschwarze Farbe, die nach einiger Zeit wieder verschwand. Danach wurde das Gemisch filtriert und vorsichtig Wasser hinzugegeben, die abgetrennte organische Phase wurde getrocknet und destilliert. Die höchstsiedenden Fraktionen wurden über Nacht in den Eisschrank bei -20 °C gestellt. Dabei fiel C₆F₁₃C₄H₈C₆F₁₃ als weißer Niederschlag aus. Dieser wurde abfiltriert und im Vakuum getrocknet.

### Darstellung mit Lithiumbuthyl

3 g C₆F₁₃C₂H₄ und 4 ml 1,6m Lithiumbuthyl in Hexan wurden in 5 ml Hexan gegeben und auf 60 °C erhitzt. Nach ca. 10 Minuten begann ein weißer Niederschlag auszufallen. Die Temperatur wurde noch für 50 Minuten belassen. Danach wurde vorsichtig Wasser zugegeben und die Phasen getrennt. Die organische Phase wurde getrocknet und destilliert. Danach wurde analog der Umsetzung mit Magnesium verfahren. Das Produktionsverhältnis von R_{F}R_{H}R_{H}R_{F} zu R_{F}R_{H}-Bu wurde mittels GC bestimmt. Als Standard wurde Perfluordekalin verwendet. Die Gesamtausbeute betrug 3,1 g (85%).
Die Verbindungen wurden durch Vergleich mit den bekannten Substanzen mittels GC, MS und ¹H-NMR identifiziert.

### Beispiel 9

Nach [4] erfolgt die Synthese von F(CF₂)₁₂(CH₂)ₙH (n = 4, 6, 8, 10, 12, 14, 16, 18, 20), F(CF₂)₁₀(CH₂)₈(CF₂)₁₀F und F(CF₂)₁₂(CH₂)₁₀(CF₂)₁₂F, indem durch radikalische Addition Perfluordecyliodid oder Pefluordodecyliodid mit den entsprechenden Alkenen oder Dialkenen umgesetzt und das entsprechende Perfluoralkyliodid abschließend mit Tributylzinnhydrid und AIBN in Toluol reduziert wird.

### Beispiel 10

Nach [6] wird analog Beispiel 9 zunächst das Perfluoralkyliodid gebildet, danach erfolgt aber die Reduktion zum semifluorierten Alkan, R_{F}R_{H} bzw. R_{F}R_{H}R_{F}, mittels Palladiumkohle oder Platinoxid als Katalysator mit Wasserstoff bei 4 bar im Autoklaven.

## Patentansprüche

1. Verwendung eines semifluorierten Alkans der allgemeinen Formeln
R_{F}R_{H} oder R_{F}R_{H}R_{F},
wobei
- R_{F} eine lineare oder verzweigte Perfluoralkyl-Gruppe und R_{H} eine lineare oder verzweigte, gesättigte Kohlenwasserstoff-Alkyl-Gruppe ist,
- das unverzweigte semifluorierte Alkan die Formel
F(CF₂)ₙ(CH₂)ₘH
oder
F(CF₂)ₙ(CH₂)ₘ(CF₂)ₙF
mit n = 3 - 20
m = 3 - 20
besitzt,
- das verzweigte semifluorierte Alkan innerhalb der Perfluoralkyl-Gruppen -FCX- -Einheiten
mit X C₂F₅, C₃F₇ oder C₄F₉
sowie innerhalb der Alkyl-Gruppen -HCY- -Einheiten
mit Y = C₂H₅, C₃H₇ oder C₄H₉
enthält, und
- stets die Gesamtzahl der Kohlenstoffatome im Perfluoralkyl-Teil bzw. in den PerfluoralkylTeilen in den Grenzen von
n = 3 - 20 sowie im Kohlenwasserstoff-Alkyl-Teil die Zahl der Kohlenstoffatome in den Grenzen von
m = 3 - 20 bleibt, zur Herstellung eines Intubations-Flüssigkeits-Beatmungsmittels.

2. Verwendung nach Anspruch 1, wobei innerhalb der Perfluoralkyl-Kette eine -CX₂- -Gruppe, sowie innerhalb einer Kohlenwasserstoff-Alkyl-Kette eine -CY₂--Gruppe enthalten ist.

3. Verwendung nach Anspruch 1 oder 2, wobei endständig im Molekül anstelle der Perfluoralkyl-Gruppe F₃C- eine FCX₂- oder F₂CX- -Gruppe gebunden ist und ebenso endständig im Molekül anstelle der Alkyl-Gruppe H₃C- eine HCY₂- oder H₂CY- -Gruppe gebunden ist.

4. Verwendung nach einem der Ansprüche 1 bis 3, **gekennzeichnet durch** wenigstens eine der Verbindungen C₆F₁₃C₈H₁₇, C₄F₉C₅H₁₁ oder C₃F₇C₄H₈C₃F₇.

## Claims

1. Use of a semi-fluorinated alkane of the general formulae
R_{F}R_{H} or R_{F}R_{H}R_{F}
wherein
- R_{F} is a straight-chain or branched perfluoroalkyl group and R_{H} is a straight-chain or branched saturated hydrocarbon alkyl group,
- the unbranched semi-fluorinated alkane is of the formula
F(CF₂)ₙ(CH₂)ₘH
or
F(CF₂)ₙ(CH₂)ₘ (CF₂)ₙF
with n = 3 - 20
m = 3 - 20,
- the branched semi-fluorinated alkane includes within the perfluoroalkyl groups -FCX- -units
with X = C₂F₅, C₃F₇ or C₄F₉
and within the alkyl groups -HCY- -units
with Y = C₂H₅, C₃H₇ or C₄H₉, and
- the total number of carbon atoms in the perfluoroalkyl part or the perfluoroalkyl parts always remains in the limits of n = 3 - 20 and in the hydrocarbon alkyl part the number of carbon atoms remains in the limits of m = 3 - 20, for the production of an intubation liquid respiration agent.

2. Use according to claim 1 wherein a -CX₂- - group is contained within the perfluoroalkyl chain and a -CY₂- - group is contained within a hydrocarbon alkyl chain.

3. Use according to claim 1 or claim 2 wherein an FCX₂- or F₂CX- - group is bound in the molecule at the end instead of the perfluoroalkyl group F₃C- and an HCY₂- or H₂CY- -group is likewise bound in the molecule at the end instead of the alkyl group H₃C.

4. Use according to one of claims 1 to 3 **characterised by** at least one of the compounds C₆F₁₃C₈H₁₇, C₄F₉C₅H₁₁ or C₃F₇C₄H₈C₃F₇.

## Revendications

1. Utilisation d'un alcane semi-fluoré des formules générales
R_{F}R_{H} ou R_{F}R_{H}R_{F},
dans laquelle
- R_{F} est un groupe perfluoroalkyle linéaire ou ramifié et R_{H} est un groupe hydrocarbure-alkyle saturé, linéaire ou ramifié,
- l'alcane semi-fluoré non ramifié possède la formule
F(CF₂)ₙ(CH₂)ₘH
ou
F(CF₂)ₙ(CH₂)ₘ(CF₂)ₙF
avec n = 3 - 20
m = 3 - 20,
- l'alcane semi-fluoré ramifié contient, à l'intérieur des groupes perfluoroalkyle, des unités -FCX-
avec X = C₂F₅, C₃F₇ ou C₄F₉,
ainsi que des unités -HYC- à l'intérieur des groupes alkyle,
avec Y = C₂H₅, C₃H₇ ou C₄H₉, et
- le nombre total d'atomes de carbone dans la partie perfluoroalkyle ou dans les parties perfluoroalkyle reste toujours dans les limites de n = 3 - 20, ainsi que dans la partie hydrocarbure-alkyle, le nombre d'atomes de carbone reste dans les limites de m = 3 - 20, pour la préparation d'un agent respiratoire liquide pour intubation.

2. Utilisation selon la revendication 1, dans laquelle un groupe -CX₂- est contenu à l'intérieur de la chaîne de perfluoroalkyle, ainsi qu'un groupe -CY₂- à l'intérieur d'une chaîne d'hydrocarbure-alkyle.

3. Utilisation selon la revendication 1 ou 2, dans laquelle un groupe FCX₂- ou F₂CX- est lié en fin de chaîne dans la molécule à la place du groupe perfluoroalkyle F₃C-, et également en fin de chaîne dans la molécule, un groupe HCY₂- ou H₂CY- est lié à la place du groupe alkyle H₃C-.

4. Utilisation selon l'une des revendications 1 à 3, **caractérisée par** au moins l'un des composés C₆F₁₃C₉H₁₇, C₄F₉C₅H₁₁ ou C₃F₇C₄H₉C₃F₇.
